# EUROPEAN PATENT APPLICATION

(11) **EP 3 951 389 A1**
(43) Date of publication of application: **09.02.2022**
(21) Application number: 20781295.9
(22) Date of filing: 27.03.2020
(51) Int. Cl.: G01N 33/53, G01N 33/543, G01N 33/545

(54) **IMMUNOASSAY REAGENT AND IMMUNOASSAY METHOD**

(30) Priority: 29.03.2019 JP 2019067785
(71) Applicant: Sekisui Medical Co., Ltd., Tokyo 103-0027 (JP)
(72) Inventor: OTA, Mieko, Tokyo 103-0027 (JP); INABA, Yuuya, Tokyo 103-0027 (JP); KOBAYASHI, Hiroki, Tokyo 103-0027 (JP); YAMAMOTO, Mitsuaki, Tokyo 103-0027 (JP)
(74) Representative: Hoffmann Eitle
(86) International application number: PCT/JP2020/013989
(87) International publication number: WO 2020/203755

(57) **Abstract**

The problem to be solved by the present invention is to provide a reagent that can reduce deposition of fouling in the reaction cell, by using an existing reaction cell, without affecting the composition of the reagent, in an immunoassay method using a reagent containing latex particles; and to provide an immunoassay method using the reagent.

Provided is an immunoassay reagent including a latex particle, wherein the latex particle contains a halogen atom. Also provided is an immunoassay method including contacting a latex particle with a sample to be measured in a reaction cell, wherein the latex particle contains a halogen atom.

## Description

### Technical Field

The present invention relates to an immunoassay reagent each including latex particles, and to an immunoassay method using the immunoassay reagent. It particularly relates to an immunoassay reagent each including latex particles which contain a halogen atom, and to an immunoassay method using the immunoassay reagent.

### Background Art

Homogeneous measurement methods using insoluble carrier particles, especially a latex agglutination immunoassay (LTIA) method, enable highly sensitive detection, and therefore, the range of analyte is expanding in the field of clinical examination. The analytes to be measured by the LTIA method include proteins, sugars, lipids, enzymes, hormones, inorganic ions and disease markers contained in biological samples such as blood and urine, and most of them have been measured with an autoanalyzer. Examples of the known autoanalyzer include a device for measuring the absorbance of a reaction solution obtained by mixing a biological sample and one or more reagents, for example, in a reaction cell; and for determining the presence or absence of a predetermined substance and the concentration thereof.

In recent years, the number of measurement items in clinical examinations has increased dramatically, as medical diagnostic technologies have been improved. Along with this, in the clinical examinations, the measurement using an autoanalyzer is required to be performed with high sensitivity for a small amount of specimens, because the amount of specimens to be distributed to each of the measurement items is limited.

Reaction cells are generally formed of hydrophobic resins. Therefore, biological samples and examination reagents easily deposit on the inner surface of the reaction cells (hereinafter "on the inner surface of the reaction cells" may be referred to simply as "in the cell"), and the resulting fouling may not be sufficiently removed only by routine cleaning procedures. In particular, when a large number of specimens are continuously processed by an automatic analyzer, the fouling may not be completely removed only by programmed cleaning procedures depending on the type of the specimens. Further, when small reaction cells are used in order to reduce the amount of the specimen, it is expected that even a slight amount of fouling residues will even reduce the sensitivity of detection by optical means to cause trouble.

Therefore, in order to analyze a small amount of specimen with high sensitivity, it is required to reduce the deposition of fouling derived from the specimens or reagents as much as possible.

For example, Patent Literature 1 discloses a reaction cell for autoanalyzers, wherein the deposition of air bubbles and contaminants is reduced by forming a mixture of
a first polymeric material that is a polyolefin resin with
a second polymeric material having at least one oxygen-containing functional group selected from the group consisting of a hydroxyl group, an ether group, a carbonyl group, a carboxyl group and an ester group.

Further, Patent Literature 2 discloses a process for reducing the deposition of contaminants on a reaction cell of an autoanalyzer by supplying an antifouling solution composed of an aqueous solution of polyethylene glycol (PEG) or polyvinylpyrrolidone (PVP), which is a water-soluble resin, to the reaction cell to form an antifouling film on the inner wall surface of the reaction cell.

### Citation List

### Patent Literature

Patent Literature 1: Japanese Patent Laid-Open No. 2018-25415
Patent Literature 2: Japanese Patent Laid-Open No. 2016-50796

### Summary of Invention

### Technical Problem

For Patent Literature 1 described above, a special reaction cell needs to be provided, and a cell attached to an existing continuous measurement device cannot be used. Further, for Patent Literature 2, a special antifouling solution is required, and therefore, it is necessary to consider the influence on the immune reaction and the change in the reagent composition.

The problem to be solved by the present invention is to provide a reagent that can be used in an immunoassay method using a reagent containing latex particles in which an existing reaction cell can be used and that can reduce deposition of fouling in the reaction cell without affecting the composition of the reagent, and to provide an immunoassay method using the reagent.

### Solution to Problem

As a result of diligent studies to solve the above problems, it has been found that the deposition of fouling derived from specimens or reagents can be reduced by incorporating a halogen atom in latex particles, to complete the present invention. That is, the present invention includes:
[1] An immunoassay reagent including latex particles, wherein the latex particles each comprise a halogen atom.
[2] The immunoassay reagent according to [1], wherein the halogen atom is fluorine.
[3] The immunoassay reagent according to [1] or [2], wherein the latex particles are polystyrene latex particles.
[4] The immunoassay reagent according to any of [1] to [3], wherein the latex particles are non-sensitized particles that are not sensitized to either antigens or antibodies.
[5] The immunoassay reagent according to any of [1] to [4], wherein the immunoassay reagent is a reagent for autoanalyzers.
[6] A reagent for measuring HbA1c comprising at least: latex particles each comprising a halogen atom; and an antibody against HbA1c.
[7] A latex immunoassay reagent for measuring HbA1c including:
   (1) a first reagent including latex particles each comprising a halogen atom; and
   (2) a second reagent including an anti-HbAlc monoclonal antibody.
[8] An immunoassay method including: contacting latex particles with a sample to be measured in a reaction cell, wherein the latex particles each contain a halogen atom.
[9] The immunoassay method according to [8], wherein the halogen atom is fluorine.
[10] The immunoassay method according to [8] or [9], wherein the latex particles are polystyrene latex particles.
[11] The immunoassay method according to any of [8] to [10], wherein the latex particles are non-sensitized particles, the latex particles are not sensitized to either antigens or antibodies.
[12] The immunoassay method according to any of [8] to [11], including performing the measurement using an autoanalyzer.
[13] A method for measuring HbA1c, comprising a step of contacting latex particles each comprising a halogen atom with an antibody against HbA1c and a sample in a reaction cell.
[14] A method for measuring HbA1c including:
   (1) a step of contacting latex particles each containing a halogen atom with a sample in a reaction cell to adsorb HbA1c contained in the sample to the latex particles; and
   (2) a step of contacting the HbA1c adsorbed to the latex particles with an anti-HbAlc monoclonal antibody to agglutinate the latex particles.
[15] A method for suppressing fouling in a reaction cell in an immunoassay method including a step of contacting latex particles with a sample in the reaction cell, wherein the latex particles each comprise a halogen atom.
[16] A latex particle used for immunoassay, wherein the particle comprises a halogen atom.

### Advantageous Effects of Invention

The present invention can provide a measurement reagent which does not easily deposit fouling derived from specimens or reagents in the reaction cell, by utilizing a conventional reaction cell as it is, and without changing the reagent composition.

According to the reagent of the present invention, the fouling does not deposit in the cell even if repeated measurements are performed, and the blank value can be prevented from becoming high. Therefore, the reagent of the present invention is particularly suitably used in autoanalyzers that measure a large number of various specimens for a large number of various examination items at one time.

### Description of Embodiments

The present invention relates to an immunoassay reagent including latex particles, characterized in that the latex particles each contain a halogen atom: and to an immunoassay method including a step of contacting latex particles containing a halogen atom with a sample in a reaction cell. Since the immunoassay method can suppress fouling in a reaction cell, the present invention relates to a method for suppressing fouling in a reaction cell in an immunoassay method, including: contacting latex particles each containing a halogen atom with a sample in the reaction cell. Hereinafter, the present invention will be described in detail.

### (Reaction cell)

The reaction cell according to the present invention is a reaction cell for holding a mixture of a specimen sample and a reagent and for performing optical measurement of the mixture, and is made of glass or plastic. Examples of the cells made of plastic include cells made of a material such as polyethylene, polypropylene, a copolymer of polyethylene and polypropylene, or polymethylmethacrylate. The reaction cell includes a nondisposable type that can be used continuously; a disposable type that is replaced after each measurement; and a semi-disposable type that is discarded, for each type of specimen to be measured, after each measurement item or after used a certain number of times or a certain period of time. The reagent of the present invention is preferably used in a reaction cell of a nondisposable type that can be used continuously or that of a semi-disposable type.

According to the present invention, the analyte in a sample may be measured manually or using a device such as a measurement device. The measurement device may be a general-purpose autoanalyzer or a dedicated measurement device (dedicated machine).

The autoanalyzers as used in the present invention refers to those manufactured and sold by companies mainly for the purpose of use in clinical examinations. Specific examples of the autoanalyzers include: so-called general-purpose reagent-type automatic analyzers such as those of automatic analyzer series manufactured by Hitachi High-Tech Corporation, those of TBA series manufactured by Toshiba Medical Systems Corporation, those of BM series manufactured by JEOL Ltd., those manufactured by Beckman Coulter Biomedical GmbH and those manufactured by Sekisui Medical Co., Ltd.; so-called dedicated reagent-type autoanalyzers such as a nearinfrared measurement device LPIA (registered trademark) (manufactured by Mitsubishi Chemical Medience Corporation) and a scattered light intensity measurement device (manufactured by Dade Behring Inc.); and blood coagulation measurement devices capable of optical measurement.

For these autoanalyzers, the measurement of a specimen is usually performed by the following procedures. An example of using an examination reagent (two-component reagent) consisting of two reagents, which is suitable for the present invention, will be described in the order of the measurement steps. The following measurement steps are performed sequentially: cleaning a reaction cell; measuring a water blank; dispensing a sample and a first reagent; mixing; dispensing a second reagent; mixing; reacting; measuring the optical change; aspirating a reaction solution; and cleaning the reaction cell. Although the measurement steps of the autoanalyzer include the step of cleaning a reaction cell as described above, such a step alone could not sufficiently remove fouling derived from the sample when using a conventional reagent.

As a dedicated measurement device, a measurement device described below, in which a sample and a reagent are mixed through a microchannel and allowed to react to perform detection, may be used. Specifically, a reaction cassette that is rotated around a horizontal rotation axis is used. This reaction cassette is provided with a microchannel and an injection hole that communicates with the microchannel and introduces a liquid sample into the microchannel. The measurement device is provided with a means of easily introducing a diluent.

The reaction channel is provided with: a reagent zone having an analytical reagent incorporated therein; and a means of disturbing the gravitational flow of the liquid sample along the microchannel by contacting the liquid sample with the analytical reagent, and stirring the liquid sample together with the analytical reagent to sufficiently promote a predetermined reaction.

The measurement device is configured so as to have
the reaction cassette above is rotated and shaken to flow a liquid sample through the microchannel and thus contact the liquid sample with an analytical reagent;
the liquid sample is stirred together with the analytical reagent to promote a predetermined reaction; and
a detectable reaction in the liquid sample is measured.

### (Halogen atom-containing latex particles)

The halogen atom-containing latex particles of the present invention are composed of polymer-based latex particles each containing a halogen atom in the molecule. Examples of the polymer constituting the polymer-based latex particles include, but are not particularly limited to, polystyrene, a styrene-styrene sulfonate copolymer, poly(vinyl naphthalene), a styrene-vinylnaphthalene copolymer, poly(methacrylic acid), poly(acrylic acid), poly(itaconic acid), a styrene-hydrophilic carboxy monomer copolymer, a styrene-methacrylic acid copolymer, a styrene-acrylic acid copolymer, and a styrene-itaconic acid copolymer. Among them, a styrene-styrene sulfonate copolymer and a styrene-vinylnaphthalene copolymer are preferable. A styrene-styrene sulfonate copolymer is particularly preferable. The halogen atom-containing latex particles of the present invention can be prepared by partially using a monomer containing a halogen atom (halogen atom-containing monomer) as a raw material of the polymer.

Examples of salts in the styrene sulfonate salt used in the present invention include, but are not particularly limited to, its sodium salt, potassium salt, lithium salt and ammonium salt. These may be used alone or in combination of two or more thereof. Among them, sodium styrene sulfonate is preferably used.

Polymer-based latex particles each containing a halogen atom in the molecule of the polymer used in the present invention can be prepared by any known method, to which the method is not particularly limited. For example, a soap-free emulsion polymerization process that does not use an emulsifier (a surfactant) can be preferably used. Examples of the polymerization initiator used in the soap-free emulsion polymerization process include potassium persulfate and ammonium persulfate, and potassium persulfate is preferable. According to the present invention, the latex particles can be prepared by charging, into a reaction vessel, ionexchanged water, a monomer as a base for particles, a halogen-containing monomer and a polymerization initiator; carrying out nitrogen gas substitution in the reaction vessel while stirring; and then performing the polymerization reaction.

The halogen atom-containing monomer is not particularly limited as long as it is a compound having a double bond or a triple bond and a halogen atom, and examples thereof include a halogen-containing styrene monomer. Examples thereof include 2-fluorostyrene, 3-fluorostyrene, 4-fluorostyrene, 2,3,4,5,6-pentafluorostyrene, 2-chlorostyrene, 3-chlorostyrene, 4-chlorostyrene, 2-bromostyrene, 3-bromostyrene, and 4-bromostyrene. Among them, 2-fluorostyrene, 3-fluorostyrene, 4-fluorostyrene and 2,3,4,5,6-pentafluorostyrene are preferred, and 4-fluorostyrene and 2,3,4,5,6-pentafluorostyrene are particularly preferred.

For the lower limit of the halogen atom content, the weight of the monomer containing a halogen atom is desirably 1% or more, based on the total weight of the monomer because when the halogen atom content is less than 1%, the effect of suppressing fouling in the reaction cell is poor. The lower limit of the halogen atom content is more preferably 2% or more, still more preferably 3% or more and most preferably 5% or more. In some cases, 6% or more, 7% or more, 8% or more, 9% or more, and 10% or more are preferable. For the upper limit of the halogen atom content, the weight of the monomer containing a halogen atom is desirably 50% or less, based on the total weight of the monomer because when the halogen atom content is more than 50%, adsorption of HbA1c on the surface of latex particles is inhibited, leading to a decrease in sensitivity. The upper limit of the halogen atom content is more preferably 40% or less, still more preferably 30% or less and most preferably 25% or less.

As a preferred range of the halogen atom content, a combination of the above lower limit and upper limit can be mentioned. Specifically, the range is 1 to 50%, more preferably 2 to 40%, still more preferably 3 to 30% and most preferably 5 to 25%.

Specific examples of the method for preparing fluorine-containing styrene particles include a method including: adding, into a vessel, styrene monomers, thus,4-fluorostyrene or 2,3,4,5,6-pentafluorostyrene and sodium styrene sulfonate, and potassium persulfate; carrying out nitrogen gas substitution in the vessel; and then performing the polymerization reaction.

The size of latex particles can be selected in the range of 0.05 to 1 µm appropriately depending on the immunoassay method of the present invention and the detection principle of the reagent, but the average particle size of 0.1 to 0.4 µm is widely used for optical measurement in autoanalyzers, and preferably the average particle size is 0.1 to 0.2 µm.

It is not clear why the deposition of fouling in the cell can be reduced in the present invention by incorporating a halogen atom in the latex particles. However, in the field of resins, polytetrafluoroethylene (PTFE) has the property that materials do not easily deposit thereon as compared with a polyethylene resin, wherein PTFE is obtained by using, as a monomer, tetrafluoroethylene in which every hydrogen atom of ethylene is replaced with a fluorine atom. When the latex particles each contain a halogen atom, it is considered that the deposition of fouling derived from specimens or reagents to the cell can be prevented for the similar reason.

### (Materials with high affinity)

An analyte material in a sample can be measured by using the reagent including latex particles of the present invention in combination with a material with high affinity for the analyte material. The materials with high affinity include proteins, peptides, amino acids, lipids, sugars, nucleic acids and haptens. They are not particularly limited by the molecular weight and whether they are naturally or synthetically derived, but polyclonal antibodies or monoclonal antibodies (including functional fragments thereof) or antigens are generally utilized. It is also possible to use, as an antibody of the present invention, a functional fragment of an antibody having an antigen-antibody reaction activity, in addition to the entire antibody molecule.

The antibody may be
an antibody obtained through the process of immunizing a general animal (such as mouse, goat or sheep) with an immunogen (an analyte material); as well as
an antibody the amino acid sequence of which has been changed, by gene recombination technology or the like, into an amino acid sequence of an animal species different from the animal which immunize the immunogen (such as a chimeric antibody, a humanized antibody or a fully humanized antibody). Examples of the functional fragment of the antibody include a fragment having an antigen-antibody reaction activity, such as F(ab')2 or Fab', or a single chain antibody (scFv). These functional fragments of antibodies can be produced by treating the antibodies obtained as described above with a proteolytic enzyme (such as pepsin or papain).

These antibodies and antigens can be incorporated in the reagent in a free state or in a state of being bound to the surface of the latex particles. Latex particles in a state in which a material with high affinity such as an antigen or an antibody has not bound thereto are sometimes referred to as non-sensitized latex particles in the present invention. The method for binding a material with high-affinity to latex particles may be any method using any binding manner including physical adsorption, chemical binding, affinity binding, or the like.

### (Available measurement principles)

The reagent including latex particles of the present invention can be suitably used for various methods which utilize biological reactions, such as enzyme immunoassay, fluorescence immunoassay, latex immunoagglutination and immunochromatography, and among them, can be more suitably used for latex immunoagglutination by measuring the degree of latex agglutination.

The latex agglutination can be measured by observing the degree of agglutination optically or electrochemically, and the concentration of an analyte can be thereby measured. Examples of the method for optical observation include a method of measuring the scattered light intensity, absorbance or transmitted light intensity with an optical instrument (such as an endpoint method or a rate method).

The measured value such as absorbance obtained by measuring a sample can be compared with the measured value such as absorbance obtained by measuring a standard substance to calculate the concentration (quantitative value) of the analyte material contained in the sample.

The measurement of absorbance of transmitted light or scattered light or the like may be either onewavelength measurement or two-wavelength measurement (difference or ratio between two wavelengths). The measurement wavelength is generally selected from 400 nm to 800 nm.

### (Samples to be measured and analytes)

For the measurement reagent and the measurement method of the present invention, the "sample" to be detected may be any sample containing an object capable of being detected by utilizing an immune reaction, and mainly includes body fluids derived from living organisms. Specific examples include whole blood, plasma, serum, blood cells, pharyngeal swab and urine.

These samples may be used as they are, but they may also be used after diluted with a specimen diluent or subjected to other pretreatments.

Examples of the analytes include proteins, peptides, amino acids, lipids, sugars, nucleic acids and haptens, but may be not limited as long as they are molecules which can be theoretically measured. Examples of the analytes include HbA1c, CRP (C reactive protein), Lp (a), MMP3 (matrix metalloproteinase 3), anti-CCP (cyclic citrullinated peptide) antibody, anti-phospholipid antibody, RPR, collagen type IV, PSA, BNP (brain natriuretic peptide), NT-proBNP, insulin, microalbumin, cystatin C, RF (rheumatoid factor), CA-RF, KL-6, PIVKA-II, FDP, D dimer, SF (soluble fibrin), TAT (thrombinantithrombin III complex), PIC, PAI, factor XIII, pepsinogen I/II, phenytoin, phenobarbital, carbamazepine, valproic acid and theophylline.

### (Immunoassay reagent, immunoassay reagent kit)

The immunoassay reagent of the present invention includes at least the above-mentioned latex particles containing a halogen atom, and also includes any other component necessary for immunoassay. It is usually provided in the form of a first reagent or a second reagent, and the latex particles can be included in the first reagent or the second reagent. In the case of non-sensitized latex particles, it is generally suitable that the latex particles are included in the first reagent, and the material with high affinity that binds to the analyte is included in the second reagent. In the case of sensitized latex particles, it is suitable that a buffer is included in the first reagent, and the latex particles are included in the second reagent. The measurement reagent of the present invention may also be composed of three reagents (three-step method) in addition to the above two reagents (two-step method). When composed of two or more reagents, the measurement reagent is also an immunoassay reagent and may also be referred to as an immunoassay reagent kit.

### (Others)

The immunoassay reagent kit of the present invention may appropriately include a buffer component (buffer solution) in addition to the above reagent. The buffer that can be used in the present invention may be any buffer generally used. Examples of the buffer include Tris-hydrochloric acid, boric acid, phosphoric acid, acetic acid, citric acid, succinic acid, phthalic acid, glutaric acid, maleic acid, glycine and salts thereof, as well as Good's buffers such as MES, Bis-Tris, ADA, PIPES, ACES, MOPSO, BES, MOPS, TES and HEPES.

The kit can also include reagents necessary for detection, a diluent for a specimen, a tool for collecting a specimen, an instruction manual, and the like.

### (Reagent for measuring HbA1c)

As described above, the present invention can be applied to any analytes, but a reagent for measuring HbA1c in blood will be described by example. The reagent for measuring HbA1c is composed of at least:
(a) latex particles each containing a halogen atom; and
(b) an antibody.

The antibody of (b) needs to include an anti-HbAlc monoclonal antibody. The antibody of (b) may include a second antibody to enhance the strength of latex agglutination. Examples of the antibody of (b) include a combination of an anti-HbAlc monoclonal antibody and a polyclonal antibody that binds to the anti-HbAlc monoclonal antibody, and a combination of an anti-HbAlc monoclonal antibody and a monoclonal antibody that reacts with the anti-HbAlc monoclonal antibody. The "polyclonal antibody that binds to an anti-HbAlc monoclonal antibody" and the "monoclonal antibody that reacts with an anti-HbA1c monoclonal antibody" are sometimes collectively referred to as "an antibody against an HbA1c monoclonal antibody".

When (b) includes one antibody, the measurement method using the above reagent can include:
a step of contacting latex particles each containing a halogen atom with a sample to adsorb HbA1c contained in the sample to the latex particles; and
a step of contacting the HbA1c adsorbed to the latex particles with the antibody to agglutinate the latex particles, to measure the HbA1c contained in the sample. When (b) includes two antibodies, the measurement method using the above reagent can include:
a step of contacting latex particles each containing a halogen atom with a sample to adsorb HbA1c contained in the sample to the latex particles;
a step of contacting the HbA1c adsorbed to the latex particles with a first antibody to form a complex of the first antibody, the HbA1c and latex particles; and
a step of contacting the complex with a second antibody to agglutinate the latex particles, to measure the HbA1c contained in the sample.

Hereinafter, the present invention will be described in more detail with reference to examples, but the present invention is not limited thereto.

### Examples

### <Methods for preparing particles>

The following raw materials were used to prepare particles:
- Styrene Monomer (manufactured by NS Styrene Monomer Co., Ltd.);
- Sodium styrene sulfonate (manufactured by Tokyo Chemical Industry Co., Ltd.);
- Potassium persulfate (manufactured by Merck);
- 4-Fluorostyrene (manufactured by Tokyo Chemical Industry Co., Ltd.);
- 2,3,4,5,6-Pentafluorostyrene (manufactured by Tokyo Chemical Industry Co., Ltd.); and
- 1-Vinylnaphthalene (manufactured by Tokyo Chemical Industry Co., Ltd.)

### [Preparation Example of particles of Comparative Example 1] Preparation of styrene particles

Into a glass reaction vessel (capacity: 2L) provided with a stirrer, a reflux condenser, a temperature detector, a nitrogen inlet tube and a jacket, 800 g of ultrapure water, 38 g of styrene monomer, 0.02 g of sodium styrene sulfonate and 0.3 g of potassium persulfate were charged. After nitrogen gas substitution in the vessel, polymerization was carried out for at 70°C 24 hours while stirring at a rate of 120 rpm.

After completion of polymerization, the resulting solution was filtered through a filter paper to collect latex particles.

Thereafter, the collected latex particles were subjected to dialysis treatment with a dialysis membrane for 48 hours to obtain purified latex particles. The particle size of the obtained latex particles was 0.119 µm.

### [Preparation Example of particles of Comparative Example 2] Preparation of vinylnaphthalene particles

Latex particles were obtained in the same manner as in Preparation Example of particles of Comparative Example 1, except for using 800 g of ultrapure water, 28.5 g of styrene monomer, 14 g of 1-vinylnaphthalene, 0.01 g of sodium styrene sulfonate and 0.2 g of potassium persulfate. The particle size of the obtained latex particles was 0.114 µm.

### [Preparation Example of particles of Example 1] Preparation of fluorine-containing particles

Latex particles were obtained in the same manner as in Preparation Example of particles of Comparative Example 1, except for using 800 g of ultrapure water, 38 g of styrene monomer, 11.1 g of 4-fluorostyrene, 0.02 g of sodium styrene sulfonate and 0.3 g of potassium persulfate. The particle size of the obtained latex particles was 0.104 µm. The amount of substance of the monomers containing fluorine was 20% based on the total amount of substance of monomers.

### [Preparation Example of particles of Example 2] Preparation of fluorine-containing particles

Latex particles were obtained in the same manner as in Preparation Example of particles of Comparative Example 1, except for using 800 g of ultrapure water, 38 g of styrene monomer, 17.7 g of 2,3,4,5,6-pentafluorostyrene, 0.02 g of sodium styrene sulfonate and 0.3 g of potassium persulfate. The particle size of the obtained latex particles was 0.107 µm. The amount of substance of the monomers containing fluorine was 20% based on the total amount of substance of monomers.

### [Preparation Example of particles of Example 3] Preparation of fluorine-containing particles

Latex particles were obtained in the same manner as in Preparation Example of particles of Comparative Example 1, except for using 800 g of ultrapure water, 38 g of styrene monomer, 2.3 g of 4-fluorostyrene, 0.02 g of sodium styrene sulfonate and 0.3 g of potassium persulfate. The particle size of the obtained latex particles was 0.107 µm. The amount of substance of the monomers containing fluorine was 5% based on the total amount of substance of monomers.

### [Preparation Example of particles of Example 4] Preparation of fluorine-containing particles

Latex particles were obtained in the same manner as in Preparation Example of particles of Comparative Example 1, except for using 800 g of ultrapure water, 38 g of styrene monomer, 5.0 g of 4-fluorostyrene, 0.02 g of sodium styrene sulfonate and 0.3 g of potassium persulfate. The particle size of the obtained latex particles was 0.110 µm. The amount of substance of the monomers containing fluorine was 10% based on the total amount of substance of monomers.

An anti-human HbA1c monoclonal antibody and a rat anti-mouse IgG monoclonal antibody were produced by a conventional method.

### [Example] Preparation of reagents

The compositions of the first reagent and the second reagent of each of Comparative Example 1 and Examples 1 to 6 are shown below. The type and content of latex particles in each first reagent are as shown in Table 1.

### (1-1) First reagent

Buffer, pH 7.0
Latex particles (*1)
0.05% ProClin 300
10% glycerol
(*1): shown in Table 1.

### (1-2) Second reagent

Buffer, pH 7.0
500 mM Sodium chloride
0.09% Sodium azide
0.05% Tween-20
0.1 mg/mL Anti-human HbA1c monoclonal antibody
0.1 mg/mL Rat anti-mouse IgG monoclonal antibody

**[Table 1]**

| Reagent | Particles | Content of particles in first reagent (% by weight) |
|---|---|---|
| Comparative Example 1 | Particles of Comparative Example 1 (styrene particles) | 0.1% |
| Comparative Example 2 | Particles of Comparative Example 2 (vinylnaphthalene particles) | 0.1% |
| Example 1 | Particles of Example 1 (Fluorine content(*): 20%) | 0.1% |
| Example 2 | Particles of Example 2 (Fluorine content(*): 20%) | 0.1% |
| Example 3 | Particles of Example 3 (Fluorine content(*): 5%) | 0.1% |
| Example 4 | Particles of Example 4 (Fluorine content(*): 10%) | 0.1% |
| Example 5 | Particles of Example 1 (Fluorine content(*): 20%) | 0.17% |
| Example 6 | Particles of Example 1 (Fluorine content(*): 20%) | 0.25% |

| | | |
|---|---|---|
| (*) Percentage (%) of the amount of substance of fluorine-containing monomer based on the total amount of substance of monomers. | | |

### [Test Example 1] Evaluation of cell fouling

The degree of fouling of a cell was evaluated when blood samples were subjected to continuous measurement using each of the reagents listed above.

### (1) Test method

For each reagent of Comparative Example 1, Example 1 and Example 2, three unused reaction cells (cell material: plastic; manufactured by Hitachi High-Tech Fielding Corporation; product name: Reaction Cell; product code: 21003) were used. The measurement sample was continuously measured 30 times for each reaction cell, and the degree of fouling in the reaction cell after the measurement was evaluated.

### (i) Measurement device and measurement wavelength Hitachi 7170 autoanalyzer, 340 nm

### (ii) Measurement sample

Blood specimen: Human blood collected with an EDTA blood collection tube was centrifuged at 2000 ×g for 5 minutes. The obtained blood cells in the lower layer were diluted by 100 times with purified water and used as a specimen.

### Blank specimen: purified water.

### (iii) Measurement procedure

Each measurement sample (6.3 µL) and a first reagent (150 µL) were charged into the reaction cell and allowed to react at 37°C for 5 minutes, and thereafter, a second reagent (50 µL) was further charged into the reaction cell and allowed to react at 37°C for 5 minutes.

For the reaction, the measurement was performed using each of the reagents so that the measurement was continuously performed in the cell with a particular number assigned.

Before and after continuous measurement of each measurement sample, the cell blank value at 340 nm was measured to calculate an increment in the cell blank value. The increment in the cell blank value calculated in the continuous measurement of the blank (purified water is used as a measurement sample and purified water is also used as the first reagent and the second reagent) as a measurement sample was subtracted from the increment in the cell blank value calculated in the continuous measurement of a specimen as a measurement sample. The value obtained by the subtraction was evaluated as a cell fouling value (mAbs.). That is, the cell fouling value (mAbs.) can be calculated by the following equation: Cell fouling value (mAbs.) = (b2 - b1) - (a2 - a1)
Increment in the cell blank value of specimen: b2 - b1
Measurement value for the cell blank before continuous measurement: b1 (mAbs.)
Measurement value for the cell blank after continuous measurement: b2 (mAbs.)
Increment in the cell blank value for the blank (purified water): a2 - a1
Measurement value for the cell blank before continuous measurement: a1 (mAbs.)
Measurement value for the cell blank after continuous measurement: a2 (mAbs.)

The average value of three cell fouling values thus calculated was calculated, and the test results were considered.

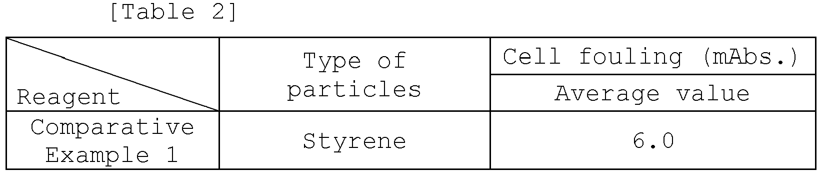

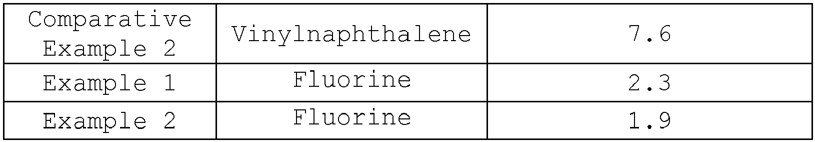

### (2) Test results

The cell fouling value after 30 continuous measurements was 6.0 mAbs. for Comparative Example 1 using styrene particles and 7.6 mAbs. for Comparative Example 2 using vinylnaphthalene particles. In contrast, for Examples 1 and 2 using fluorine-containing particles, the cell fouling values were 2.3 and 1.9 mAbs., respectively. From the results, it is considered that the reagent using the fluorine-containing particles of the present invention prevents fouling derived from blood specimens or particles from adsorbing on the inner surface of the reaction cell and the reaction cell is not easily fouled, as compared with those using the styrene particles and the vinylnaphthalene particles.

### [Test Example 2]

### (1) Test method

The measurement was continuously performed 20 times by using each reagent of Examples 1, 3 and 4 and using one cell for each reagent.

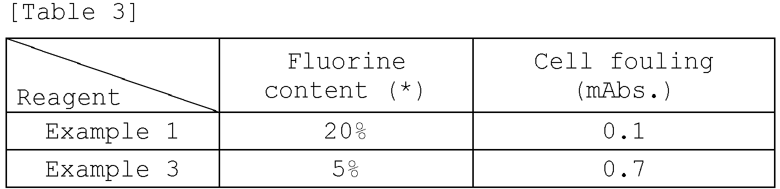

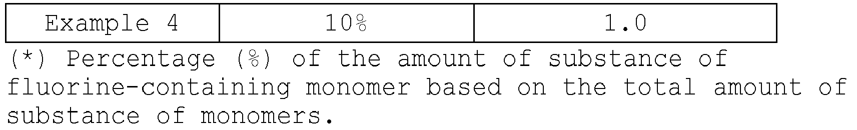

(*) Percentage (%) of the amount of substance of fluorine-containing monomer based on the total amount of substance of monomers.

### (2) Test results

The cell fouling value was 0.1 mAbs. for Example 1 using particles having the fluorine content of 20%; 0.7 mAbs. for Example 3 using particles having the fluorine content of 5%; and 1.0 mAbs. for Example 4 using particles having the fluorine content of 10%. The results show that the reagent using the fluorine-containing styrene particles of the present invention prevents the fouling derived from blood specimens or particles from adsorbing on the inner surface of the reaction cell and the reaction cell is not easily fouled, when the fluorine content of the particles is between 5 and 20%.

### [Test Example 3]

### (1) Test method

The measurement was continuously performed 30 times by using each reagent of Examples 1, 5 and 6 and using three cells for each reagent.

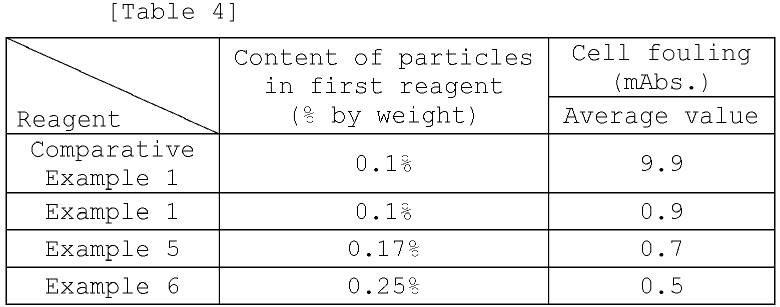

### (2) Test results

For Comparative Example 1 using the first reagent containing styrene particles, the cell fouling value was 9.9 mAbs. In contrast, in the case of the reagents using fluorine-containing particles of the present invention, the cell fouling value was 0.9 mAbs. for Example 1 wherein the fluorine content in the first reagent was 0.1%; 0.7 mAbs. for Example 5 wherein the fluorine content in the first reagent was 0.17%; and 0.5 mAbs. for Example 6 wherein the fluorine content in the first reagent was 0.25%, indicating that in each Example, there was little cell fouling. The results show that for the reagent using fluorine-containing particles of the present invention, even if the content (% by weight) of the fluorine-containing particles contained in the first reagent is increased, the cell fouling does not increase, and the reaction cell is not easily fouled.

### Industrial Applicability

The present invention can provide a measurement reagent which can be used by utilizing a conventional reaction cell as it is; does not easily deposit fouling derived from specimens or the reagent in the reaction cell without changing the composition. Therefore, the use of the reagent of the present invention is preferable, because fouling is not deposited in the cell, even if the measurement is repeatedly performed, particularly with an autoanalyzer or the like, and the blank value can be prevented from increasing.

## Claims

1. An immunoassay reagent comprising latex particles, wherein the latex particles each comprise a halogen atom.

2. The immunoassay reagent according to claim 1, wherein the halogen atom is fluorine.

3. The immunoassay reagent according to claim 1 or 2, wherein the latex particles are polystyrene latex particles.

4. The immunoassay reagent according to any of claims 1 to 3, wherein the latex particles are non-sensitized particles that are not sensitized to either antigens or antibodies.

5. The immunoassay reagent according to any of claims 1 to 4, wherein the immunoassay reagent is a reagent for autoanalyzers.

6. A reagent for measuring HbA1c comprising at least: latex particles each comprising a halogen atom; and an antibody against HbA1c.

7. A latex immunoassay reagent for measuring HbA1c, comprising:
(1) a first reagent comprising latex particles each comprising a halogen atom; and
(2) a second reagent comprising an anti-HbAlc monoclonal antibody.

8. An immunoassay method, comprising:
contacting latex particles with a sample to be measured in a reaction cell,
wherein the latex particles each contain a halogen atom.

9. The immunoassay method according to claim 8, wherein the halogen atom is fluorine.

10. The immunoassay method according to claim 8 or 9, wherein the latex particles are polystyrene latex particles.

11. The immunoassay method according to any of claims 8 to 10, wherein the latex particles are non-sensitized particles, the latex particles are not sensitized to either antigens or antibodies.

12. The immunoassay method according to any of claims 8 to 11, comprising performing the measurement using an autoanalyzer.

13. A method for measuring HbA1c, comprising:
contacting latex particles each comprising a halogen atom with an antibody against HbA1c and a sample in a reaction cell.

14. A method for measuring HbA1c, comprising:
(1) contacting latex particles each containing a halogen atom with a sample in a reaction cell to adsorb HbA1c contained in the sample to the latex particles; and
(2) contacting the HbA1c adsorbed to the latex particles contact with an anti-HbAlc monoclonal antibody to agglutinate the latex particles.

15. A method for suppressing fouling in a reaction cell in an immunoassay method, comprising a step of contacting latex particles with a sample in the reaction cell, wherein the latex particles each comprise a halogen atom.

16. A latex particle used for immunoassay, wherein the particle comprises a halogen atom.
